# EUROPÄISCHE PATENTSCHRIFT

(11) **EP 2 054 114 B1**
(45) Veröffentlichungstag und Bekanntmachung des Hinweises auf die Patenterteilung: **11.03.2015**
(21) Anmeldenummer: 07802775.2
(22) Anmeldetag: 22.08.2007
(51) Int. Cl.: A61M 16/06

(54) **INDIVIDUELL ANGEPASSTE BEATMUNGSMASKE**
INDIVIDUALLY ADAPTED BREATHING MASK
MASQUE RESPIRATOIRE INDIVIDUELLEMENT ADAPTÉ

(30) Priorität: 23.08.2006 DE 102006039448
(43) Veröffentlichungstag der Anmeldung: 06.05.2009
(73) Patentinhaber: Nierhaus, Stefan, 45481 Mühlheim/Ruhr (DE); Jansen, Ludger, 45481 Mühlheim/Ruhr (DE)
(72) Erfinder: JANSEN, Ludger, 45481 Mülheim/Ruhr (DE)
(74) Vertreter: Cohausz & Florack
(86) Internationale Anmeldenummer: PCT/EP2007/058702
(87) Internationale Veröffentlichungsnummer: WO 2008/023028

(56) Entgegenhaltungen:
- WO-A-2004/037153
- FR-A- 2 658 725
- FR-A- 2 731 624
- FR-A- 2 749 176
- GB-A- 848 215
- GB-A- 2 397 244
- US-A- 4 677 977

## Beschreibung

Die Erfindung betrifft eine Beatmungsmaske für die Mund-Nasen- oder Nasenbeatmung, umfassend ein flexibles, an die Gesichtsanatomie des Patienten individuell angepasstes Maskenelement und wenigstens ein flächig auf dem Maskenelement außen aufliegendes Versteifungselement, wobei das Maskenelement als ein Vollkörper aus einem elastischen Material ausgebildet ist. Ferner betrifft die Erfindung ein Verfahren zur Herstellung einer solchen Beatmungsmaske

Mund-Nasen- oder Nasen-Beatmungsmasken der vorstehend beschriebenen Art sind in der Medizintechnik seit Jahren bekannt. Neben dem Einsatz in der Intensivmedizin kommen sie insbesondere bei der Klinik- und Heimbehandlung chronischer respiratorischer Erkrankungen zum Einsatz, bei denen der Patient beispielsweise nachts auf eine Beatmungshilfe angewiesen ist. Im Vordergrund steht hierbei die Behandlung der Schlafapnoe und der Heimbeatmung, z.B. bei COPD-Patienten. Beatmungsmasken der eingangs genannten Art werden aber auch bei COPD-Patienten oder auch MS-Patienten eingesetzt.

Eine aus der Praxis bekannte, individuell angepasste Mund-Nasen-Beatmungsmaske umfasst einen Silikonvollkörper als flexibles Maskenelement, welcher derart geformt ist, dass er über seine Innenseite unmittelbar an Mund und Nase des Patienten anliegt. Bei dieser Maske sind darüber hinaus an der Innenseite des Silikonvollkörpers zwei einen Beatmungsschlitz begrenzende Stege angeformt, die beim Tragen der Maske in den Mund des Patienten eingeführt werden. Ferner umfasst die vorstehend beschriebene Maske ein oberhalb der Nase und unterhalb des Mundes angeordnetes Kunststoffversteifungselement, welches außenseitig in den Silikonvollkörper der Beatmungsmaske eingelassen ist und der Befestigung von Haltebändern dient. Solche Masken weisen hinsichtlich des Tragekomforts einige gravierende Nachteile auf. So durchströmt die Beatmungsluft den Maskenvollkörper aufgrund der direkten Anlage an Mund und Nase des Patienten mit vergleichsweise hoher Geschwindigkeit, was bei der Nasenatmung langfristig zu einer Schleimhautaustrocknung führt. Aufgrund der massiven Ausführung des Maskenkörpers kommt es ferner immer wieder zu Druckstellen an Nasenwurzel und Nasenrücken. Zudem führt die unzureichende Flexibilität des Maskenkörpers bei einseitiger Belastung, wie sie beim Schlafen des Patienten auf der Seite vorkommt, zu Undichtigkeiten, denen einerseits nur durch eine Beatmungsdruckerhöhung oder durch ein Anziehen der Haltebänder, was seinerseits wieder zu stärker ausgebildeten Druckstellen führt, begegnet werden kann. Schließlich kann es durch die Auflage der Maske im Bereich der Frontzähne zu dauerhaften Schäden und/oder Druckschmerzen an den Zahnhälsen kommen. Ein weiteres Problem ist die Versorgung von Patienten mit Platzangst, die zu eng anliegende Masken nicht tolerieren.

Aus dem Stand der Technik sind eine Vielzahl von Beatmungsmasken bereits bekannt, wie beispielsweise den Druckschriften GB848215 A, US4677977 A1, GB2397244 A, FR2658725 A1, FR2749176 A1, FR2731624 A1 und WO2004/037153 A2 entnommen werden kann.

Ausgehend von diesem Stand der Technik liegt der Erfindung die Aufgabe zugrunde, eine individuell angepasste Mund-Nasen-Beatmungsmaske zu schaffen, welche einfach aufgebaut ist und sich durch hohe Dichtigkeit und dabei einen besonders hohen Tragekomfort auszeichnet.

Die Aufgabe wird erfindungsgemäß mit einer Beatmungsmaske nach Patentanspruch 1 gelöst.

Die erfindungsgemäße Beatmungsmaske umfasst ein inneres flexibles Maskenelement in Form eines aus einem elastischen Material bestehenden Vollkörpers sowie wenigstens ein außen darauf aufliegendes Versteifungselement. Dadurch ist die Maske einfach aufgebaut und somit kostengünstig herstellbar. Die erfindungsgemäße Beatmungsmaske kann als Mund-Nasen-Beatmungsmaske oder auch als reine Nasen-Beatmungsmaske ausgeführt sein. Entsprechend umschließt die umlaufende innere Dichtlippe den Mund-Nasenbereich oder ausschließlich den Nasenbereich. In beiden Fällen ist die umlaufende innere Dichtlippe ebenso wie der Vollkörper des Maskenelementes an die Gesichtsanatomie des Patienten individuell angepasst und bewirkt somit einen angenehmen, druckstellenfreien Sitz der Beatmungsmaske bei gleichzeitig exzellenter Abdichtung. Die Bezeichnung "innere Dichtlippe" ist dabei im Sinne der Erfindung derart auszulegen, dass die Dichtlippe hinsichtlich der umfangsmäßigen Außenkontur des Maskenelementes nach innen versetzt angeordnet ist.

Das wenigstens eine äußere flächig auf dem Maskenelement aufliegende Versteifungselement sorgt im Zusammenspiel mit dem Maskenelement für einen guten, dichten Sitz der Maske auch bei einseitiger Belastung, wie sie z.B. beim Schlafen des Patienten auf der Seite auftritt. Da die innere umlaufende Dichtlippe, nicht aber das Maskenelement als solches unmittelbar am Mund-Nasen- oder Nasenbereich des Patienten anliegt, ist gewährleistet, dass die bei der Nasenatmung in die Maske einströmende Luft nicht unmittelbar mit hoher Geschwindigkeit in die Nase einströmt, sondern zunächst in einem von der umlaufenden inneren Dichtlippe umschlossenen freien Beatmungsvolumen (Totraum) verwirbelt, erwärmt und befeuchtet wird, wodurch einem Austrocknen der Nasenschleimhäute wirksam begegnet wird. Im Gegensatz zu konfektionierten Beatmungsmasken wird das freie Beatmungsvolumen (Totraum) durch die Anpassung an die Gesichtsanatomie aber minimal gehalten, um eine möglichst effiziente Beatmung zu gewährleisten.

Nach einer ersten vorteilhaften Ausgestaltung der Erfindung ist das individuell an die Gesichtsanatomie des Patienten angepasste, flexible Maskenelement derart geformt, dass es den Kinnbereich des Patienten umschließt. Hierdurch ist sichergestellt, dass der Patient den Mund nicht unkontrolliert öffnen kann, was hauptsächlich bei älteren Patienten mit bereits erschlafftem Bindegewebe im Gesichtsbereich speziell in der Schlafphase zu einer Undichtigkeit führt.

Nach einer weiteren vorteilhaften Ausgestaltung der Erfindung weist das Maskenelement außenseitig eine Vertiefung zur formschlüssigen Aufnahme des Versteifungselementes auf. Hierdurch wird eine relative Positionsveränderung zwischen dem Maskenelement und dem aufliegenden wenigstens einen Versteifungselement wirksam vermieden. Ferner kann das Versteifungselement derart in das Maskenelement eingelegt werden, dass die Außenkontur des wenigstens einen Versteifungselements stufenlos in den umgebenden Rand des Maskenlements übergeht.

Nach einer weiteren vorteilhaften Ausgestaltung der Erfindung ist vorgesehen, dass das wenigstens eine Versteifungselement im Bereich des Nasenrückens eine Aufsparung aufweist. Hierdurch werden der Nasenrücken entlastet und dort entstehende Druckstellen vermieden.

Das elastische Material, aus dem das Maskenelement gefertigt ist, muss sich neben geeigneten physikalischen Eigenschaften auch durch ein hohes Maß an Hautverträglichkeit auszeichnen. Das elastische Material, aus dem das Maskenelement gefertigt ist, ist daher ein Silikon-Elastomer, insbesondere ein biokompatibles medical grade 2-Silikon-Elastomer.

Die Dichtigkeit der Beatmungsmaske und der Tragekomfort werden noch weiter gesteigert, in dem das elastische Material, aus dem das Maskenelement gefertigt ist, in den verschiedenen Bereichen des Maskenelementes eine unterschiedliche Härte aufweist. So ist es von Vorteil, dass das Material der umlaufenden inneren Dichtlippe eine geringere Härte aufweist als das Material des übrigen Maskenelements. Hierdurch wird ein besonders komfortabler und gleichzeitig dichter Sitz der Beatmungsmaske ermöglicht. Die Dichtigkeit wird durch den Luftdruck innerhalb der Maske auf die Dichtlippe zusätzlich noch verstärkt. Die Härte des Materials der umlaufenden inneren Dichtlippe beträgt dabei 10 bis 30 Shore, besonders bevorzugt 25 Shore.

Das Maskenelement umfasst ferner an seiner Außenseite ein für den Anschluss eines Beatmungsschlauches vorgesehenes Anschlussstück. Dieses weist eine gegenüber dem Material des übrigen Maskenelementes erhöhte Härte auf, so dass ein unbeabsichtigtes Abknicken des Anschlussstückes und somit eine die Beatmung behindernde Verkleinerung seines effektiven Querschnitts verhindert wird. Die Härte des Anschlussstückes beträgt 40 bis 70 Shore, insbesondere 40 Shore.

Der Erfindung liegt weiterhin die Aufgabe zugrunde, ein Verfahren zur Herstellung einer Beatmungsmaske für die Mund-Nasen- oder Nasenbeatmung der vorstehend beschriebenen Art anzugeben, welches die Herstellung der Beatmungsmasken mit hoher Präzision bei gleichzeitig niedrigen Kosten ermöglicht.

Die Aufgabe wird erfindungsgemäß durch ein Verfahren zur Herstellung einer Beatmungsmaske für die Mund-Nasen- oder Nasenbeatmung nach einem der Ansprüche 1 bis 4 gelöst, welches folgende Schritte umfasst:
- Herstellen der Innenform der Beatmungsmaske durch Abformen des Mund-Nasen- oder Nasenbereichs des Patienten,
- konturgerechtes Aufbringen eines ersten flächigen Materialstücks auf die Innenform als Platzhalter für das Maskenelement, wobei das Materialstück einen Formkörper zur Abbildung des von der Dichtlippe des Maskenelementes umschlossenen Hohlraums enthält,
- Aufbringen eines zweiten flächigen Materialstücks auf das erste Materialstück zur Erzeugung des wenigstens einen Versteifungselementes,
- Herstellung der Außenform der Beatmungsmaske durch Überbetten der Innenform,
- Entfernen des ersten flächigen Materialstücks von der Innenform, wobei das zweite flächige Materialstück als wenigstens ein Versteifungselement weiter an der Außenform anliegt,
- Erneutes Aufsetzen des den Hohlraum abbildenden Formkörpers auf die Innenform und Befüllen des Hohlraums zwischen Innenform und Außenform mit dem Material des Maskenelementes und
- Aushärten des Materials des Maskenelementes.

Gemäß dem erfindungsgemäßen Verfahren wird zunächst die anatomische Form des Mund-Nasen- oder Nasenbereiches des Patienten mit einer geeigneten Formmasse, typischerweise Silikon, abgeformt, wodurch eine Negativform des interessierenden Gesichtsbereiches entsteht. Auf Basis dieser Negativform wird ein Modell des Mund-Nasen- oder Nasenbereiches des Patienten erzeugt, welches als Innenform für die herzustellende Beatmungsmaske dient. Typischerweise ist dies ein Gipsmodell.

Sodann wird ein erstes flächiges Materialstück auf die Innenform aufgebracht, das im Herstellungsprozess als Platzhalter für das die innere umlaufende Dichtlippe umfassende Maskenelement dient und einen Formkörper zur Abbildung des von der Dichtlippe des Maskenelementes umschlossenen Hohlraums enthält. Dieses Materialstück besteht vorzugsweise aus einem weichen thermoplastischen Material, welches typischerweise bei ca. 160°C optimale Verarbeitungseigenschaften für das konturgerechte Auftragen auf die Innenform besitzt. Hierbei kann es sich beispielsweise um Ethylen-Vinylacetat-Copolymere (EVA) handeln, wie sie von der Firma ERKODENT® Erich Kopp GmbH unter der Marke Erkoflex® vertrieben werden. Dieses Material wird unter Einwirkung von Temperatur fließfähig und kann sich so konturgerecht an die darunter liegende Form anpassen. Der in dem ersten flächigen Materialstück enthaltene Formkörper besteht zweckmäßigerweise aus einer Knetmasse.

Über das erste flächige Materialstück wird ein zweites flächiges Materialstück aufgebracht, so dass dieses flächig auf dem ersten Materialstück aufliegt. Das zweite flächige Materialstück besteht zweckmäßigerweise aus einem harten thermoplastischen Material, welches unter Temperatureinwirkung ebenfalls fließfähig wird und bei Abkühlung wieder seine ursprüngliche Härte erlangt. Als hartes thermoplastisches Material wird bevorzugt ein glycolmodifiziertes Polyethylenterephtalat (PETG) eingesetzt. Dies wird u.a. unter der Marke Erkodur® von der Firma ERKODENT® Erich Kopp GmbH vertrieben. Mit dem vorstehend beschriebenen Verfahrensschritt kann somit das wenigstens eine Versteifungselement, welches erfindungsgemäß außen auf dem Maskenelement aufliegt, unmittelbar aus einem flächigen Materialstück hergestellt werden. Dabei versteht es sich, dass das zweite Materialstück ebenso wie das erste an seinem jeweiligen Umfang auf ein geeignetes Maß beschnitten wird.

Nach der Erzeugung des wenigstens einen Versteifungselementes wird die Außenform der Beatmungsmaske durch Überbetten der Innenform hergestellt. Dies erfolgt im Einzelnen derart, dass eine Formmasse, typischerweise wiederum Gips, auf die mit den vorstehend beschriebenen flächigen Materialstücken belegte Innenform aufgetragen wird, wobei die Außenform in an sich bekannter Weise mit einer entsprechenden Ausnehmung für das an dem Maskenelement vorgesehene Anschlussstück für einen Beatmungsschlauch versehen wird.

Nach Herstellung der Außenform wird sodann das als Platzhalter für das Maskenelement und die daran vorgesehene umlaufende innere Dichtlippe dienende Materialstück nebst dem den von der Dichtlippe umschlossenen Hohlraum abbildenden Formkörper entfernt, wobei das aus dem zweiten flächigen Material fertig hergestellte wenigstens eine Versteifungselement weiter an der Außenform anliegt. Nach erneutem Aufsetzen der den Hohlraum abbildenden Formmasse - hierbei kann das aus einer Knetmasse bestehende Modell durch ein Gipsmodell ersetzt werden - auf die Innenform wird die Außenform nun auf die Innenform aufgesetzt, wobei infolge der zuvor entfernten ersten und zweiten Folie zwischen den beiden Formen ein Hohlraum verbleibt, welcher nun exakt der Form des nun herzustellenden Maskenelementes samt umlaufender innerer Dichtlippe entspricht. Dieser Hohlraum wird durch ein geeignetes elastisches Material, nämlich flüssiges Silikon, gefüllt. Abschließend wird das eingefüllte Material ausgehärtet. Bevorzugt erfolgt dies bei Überdruck, insbesondere bei einem Überdruck von ca. 2 bar. Nach erfolgtem Aushärten kann sodann die Außenform abgenommen und das fertiggestellte Maskenelement nebst dem darauf aufliegenden wenigstens einen Versteifungselement entnommen werden.

Durch das vorstehend beschriebene Verfahren lassen sich individuell angepasste Beatmungsmasken für die Mund-Nasen- oder Nasenbeatmung mit hoher Präzision und vergleichsweise geringen Kosten herstellen.

Nach einer bevorzugten Weiterbildung des erfindungsgemäßen Verfahrens wird vor dem Aufbringen des ersten flächigen Materialstücks die Kontur der Dichtlippe der herzustellenden Maske in das Modell eingeformt, bevorzugt eingefräst oder einradiert. Durch diesen zusätzlichen Materialabtrag an der Innenform wird ein entsprechend größerer Hohlraum für die anschließend abzuformende umlaufende innere Dichtlippe geschaffen. Dadurch weist diese zusätzliches Material auf und liegt somit mit höherer Vorspannung am Mund-Nasen- oder Nasenbereich des Patienten an, wobei sich Vorteile für die Dichtigkeit ergeben.

Nach einer besonders vorteilhaften Ausgestaltung des erfindungsgemäßen Verfahrens ist das erste flächige Materialstück als ein erster und ein zweiter Folienabschnitt ausgebildet, wobei zunächst der erste Folienabschnitt als Platzhalter für die innere Dichtlippe konturgerecht auf die Innenform aufgebracht wird, anschließend der Formkörper auf den ersten Folienabschnitt aufgebracht wird und schließlich der zweite Folienabschnitt als Platzhalter für den Vollkörper des Maskenelementes konturgerecht auf den ersten Folienabschnitt und den Formkörper aufgebracht wird. Durch den Einsatz zweier einfacher Folienabschnitte, die nacheinander auf die Innenform aufgebracht werden, können einfache kommerziell verfügbare Materialien verwendet werden, die nicht eigens für die Aufnahme des Formkörpers vorbereitet werden müssen, da dieser einfach zwischen den Folien positioniert wird.

Bei dem ersten und dem zweiten Folienabschnitt kann es sich um Tiefziehfolienabschnitte, insbesondere Tiefziehfolienabschnitte aus einem weichen thermoplastischen Material, beispielsweise EVA, handeln. Die Dicke des zweiten Folienabschnitts liegt vorzugsweise bei 3 mm bis 4 mm, vorzugsweise ca. 3,5 mm.

Im Folgenden wird die Erfindung anhand einer ein Ausführungsbeispiel darstellenden Zeichnung näher erläutert. Es zeigen:
- Fig. 1: eine erfindungsgemäße Mund-Nase-Beatmungsmaske in Außenansicht,
- Fig. 2: die Mund-Nase-Beatmungsmaske der Fig. 1 in Seitenansicht,
- Fig. 3: das Maskenelement der Mund-Nase-Beatmungsmaske der Fig. 1 in einer Innenansicht und
- Fig. 4: die Mund-Nase-Beatmungsmaske der Fig. 1 in einer seitlichen Schnittansicht entsprechend der Schnittlinie IV-IV aus Fig. 1.

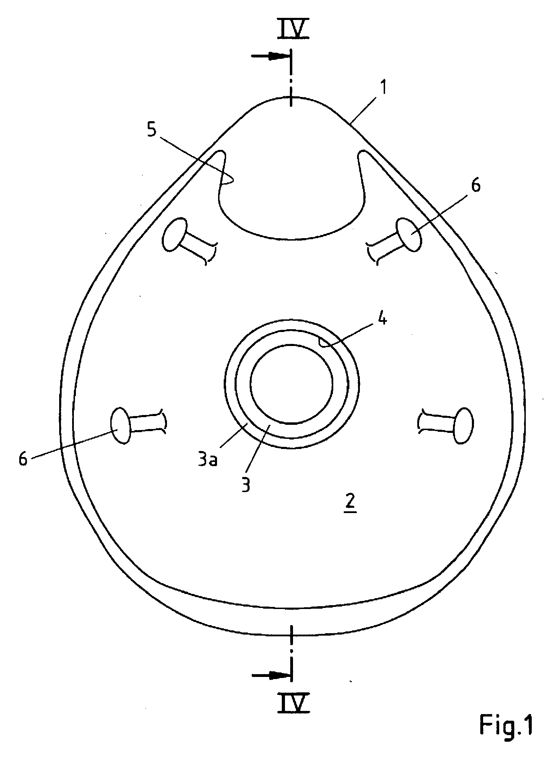

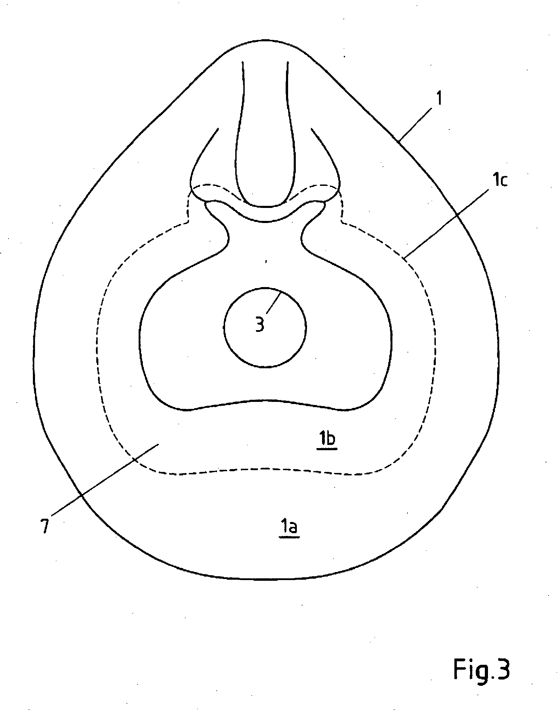

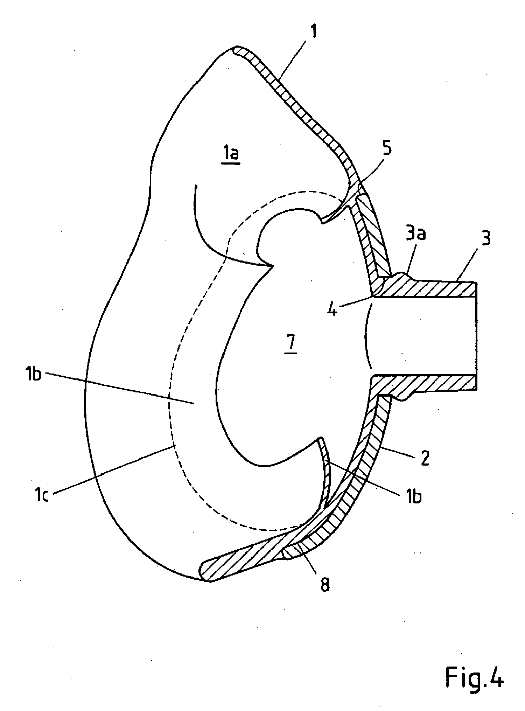

Die in der Fig. 1 in Außenansicht dargestellte erfindungsgemäße Mund-Nase-Beatmungsmaske umfasst ein flexibles Maskenelement 1 und ein auf dem Maskenelement außen aufliegendes Versteifungselement 2. Wie insbesondere aus den Fig. 3 und 4 hervorgeht, ist das Maskenelement 1 als Vollkörper 1a ausgebildet und besteht aus einem elastischen Material, vorliegend einem medical grade 2-Silikon. Das Maskenelement (1) ist an die Gesichtsanatomie des Patienten individuell angepasst und vorliegend derart geformt, dass es den Kinnbereich des Patienten umschließt. Hier durch wird erreicht, dass der Patient den Mund nicht unkontrolliert öffnen kann, was hauptsächlich bei älteren Patienten mit bereits erschlafftem Bindegewebe im Gesichtsbereich speziell in der Schlafphase zu einer Undichtigkeit führt. Das Versteifungselement 2 seinerseits besteht aus einem harten thermoplastischen Kunststoff und weist eine zentrale runde Öffnung 4 auf, durch welche ein mit einem Kragen 3a versehenes Anschlussstück 3 des Maskenelementes hindurchragt, an welches ein Beatmungsschlauch (nicht dargestellt) anschließbar ist.

Ferner umfasst das Versteifungselement 2 eine Aussparung 5, deren Funktion unten näher erläutert wird. Schließlich sind an das Versteifungselement 2 insgesamt vier Stifte 6 mit oberseitiger Verdickung angeformt, an denen Haltebänder (nicht dargestellt) befestigt werden können. Die Haltebänder werden in an sich bekannter Weise am Kopf des Patienten derart befestigt, dass die Beatmungsmaske an den Mund-Nase-Bereich des Patienten angedrückt wird.

In Fig. 3 ist nun das Maskenelement 1 der erfindungsgemäßen Beatmungsmaske in einer Innenansicht dargestellt. Wie in Fig. 3 deutlich zu erkennen, umfasst das Maskenelement 1 erfindungsgemäß eine an die Gesichtsanatomie des Patienten individuell angepasste umlaufende innere Dichtlippe 1b, welche integraler Bestandteil des Maskenelementes 1 ist und an einer Übergangskante 1c an diesem angesetzt ist. Die umlaufende innere Dichtlippe 1b umschließt dicht den Mund-Nase-Bereich des Patienten und bewirkt somit einen angenehmen, druckstellenfreien Sitz der Beatmungsmaske bei gleichzeitig exzellenter Abdichtung. Wie insbesondere der Fig. 4 zu entnehmen ist, umschließt die umlaufende innere Dichtlippe 1b einen Hohlraum 7 als freies Beatmungsvolumen. Diese hat den Vorteil, dass die in die Beatmungsmaske einströmende Luft dort zunächst verwirbelt wird, sich dabei erwärmt und befeuchtet wird, so dass bei der Nasenatmung einem Austrocknen der Nasenschleimhäute wirksam begegnet wird.

Ein druckstellenfreier Sitz der erfindungsgemäßen Beatmungsmaske insbesondere auf dem Nasenrücken des Patienten wird auch dadurch gewährleistet, dass, wie oben bereits erwähnt, das Versteifungselement 2 im Bereich des Nasenrückens des Maskenelementes 1 eine U-förmige Aussparung 5 aufweist (siehe Fig. 1), wodurch der Druck der Beatmungsmaske auf den Nasenrücken verringert wird. Hierdurch wird die Dichtigkeit der Beatmungsmaske nicht beeinträchtigt, da erfindungsgemäß der dichte Sitz der Beatmungsmaske durch die innere umlaufende Dichtlippe 1b und nicht wie im Stand der Technik durch den Vollkörper des Maskenelementes als solchen erreicht wird.

Wie insbesondere in der Schnittansicht der Fig. 4 erkennbar, liegt das äußere Versteifungselement 2 in einer im Maskenelement 1 dafür vorgesehenen Vertiefung 8, wodurch die Außenkontur des Versteifungselementes 2 im wesentlichen stufenlos in den umgebenen Rand des Maskenelements 1 übergeht. Der Vorteil ist hierbei, dass durch die Vertiefung 8 eine relative Positionsveränderung zwischen dem Maskenelement 1 und dem aufliegenden Versteifungselement 2 wirksam vermieden werden kann.

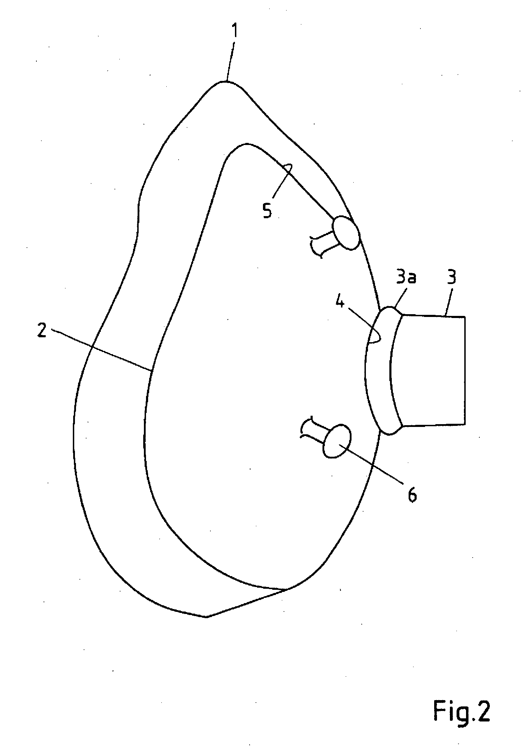

Bei der in den Fig. 1 - 4 dargestellten erfindungsgemäßen Beatmungsmaske weist das Material der umlaufenden inneren Dichtlippe 1b eine geringer Härte auf als das Material des übrigen Maskenelementes 1. Die Härte des Materials der umlaufenden inneren Dichtlippe 1b beträgt dabei 10 - 30 Shore, insbesondere 25 Shore. Ferner weist das Material des Anschlussstückes 3 für einen Beatmungsschlauch (nicht dargestellt) eine höhere Härte auf als das Material des übrigen Maskenelements 1. Die Härte des Materials des Anschlussstücks 3 beträgt 40 - 70 Shore, insbesondere 50 Shore. Die geringere Härte der umlaufenden inneren Dichtlippe 1b sorgt für einen besonders komfortablen und gleichzeitig dichten Sitz der Beatmungsmaske, während durch die höhere Härte des Anschlussstückes 3 dessen unbeabsichtigtes Abknicken wirksam verhindert wird.

Nicht dargestellt ist eine weitere für die ausschließliche Nasenbeatmung vorgesehene Ausführungsform der erfindungsgemäßen Beatmungsmaske, bei der entsprechend allein der Nasenbereich des Patienten von der inneren umlaufenden Dichtlippe umschlossen ist. Hinsichtlich der Vorteile einer solchen Nasenbeatmungsmaske gilt sinngemäß das vorstehend Gesagte.

## Patentansprüche

1. Beatmungsmaske für die Mund-Nasen- oder Nasenbeatmung, umfassend ein flexibles, an die Gesichtsanatomie des Patienten individuell angepasstes Maskenelement (1) und wenigstens ein außen auf dem Maskenelement (1) flächig aufliegendes Versteifungselement (2), wobei das Maskenelement (1) als ein Vollkörper (1a) aus einem elastischen Material ausgebildet ist, wobei
das Maskenelement (1) eine den Mund-Nasen- oder Nasenbereich umschließende, umlaufende innere Dichtlippe (1b) umfasst, und
wobei das Maskenelement (1) an seiner Außenseite ein für den Anschluss eines Beatmungsschlauches vorgesehenes Anschlussstück (3) umfasst, wobei
- das elastische Material, aus dem das Maskenelement (1) gefertigt ist, ein Silikon-Elastomer ist,
- das Material der umlaufenden inneren Dichtlippe (1b) eine geringere Härte aufweist als das Material des übrigen Maskenelements (1), wobei die Härte des Materials der umlaufenden inneren Dichtlippe (1b) 10 bis 30 Shore beträgt, und
- das Material des Anschlussstücks (3) eine höhere Härte aufweist als das Material des übrigen Maskenelements (1), wobei die Härte des Materials des Anschlussstückes (3) 40 bis 70 Shore beträgt.

2. Beatmungsmaske nach Anspruch 1,
**dadurch gekennzeichnet, dass** das flexible, an die Gesichtsanatomie des Patienten individuell angepasste Maskenelement (1) derart geformt ist, dass es den Kinnbereich des Patienten umschließt.

3. Beatmungsmaske nach Anspruch 1 oder 2,
**dadurch gekennzeichnet, dass** das Maskenelement (1) außenseitig eine Vertiefung (8) zur formschlüssigen Aufnahme des wenigstens einen Versteifungselementes (2) aufweist.

4. Beatmungsmaske nach einem der Ansprüche 1 bis 3,
**dadurch gekennzeichnet, dass** das wenigstens eine Versteifungselement (2) im Bereich des Nasenrückens eine Aussparung (5) aufweist.

5. Verfahren zur Herstellung einer Beatmungsmaske für die Mund-Nasen- oder Nasenbeatmung nach einem der Ansprüche 1 bis 4,
**gekennzeichnet durch** folgende Verfahrensschritte:
- Herstellen der Innenform der Beatmungsmaske **durch** Abformen des Mund-Nasen- oder Nasenbereichs des Patienten,
- konturgerechtes Aufbringen eines ersten flächigen Materialstücks auf die Innenform als Platzhalter für das Maskenelement (1), wobei das Materialstück einen Formkörper zur Abbildung des von der Dichtlippe (1b) des Maskenelementes (1) umschlossenen Hohlraums (7) enthält,
- Aufbringen eines zweiten flächigen Materialstücks auf das erste Materialstück zur Erzeugung des wenigstens einen Versteifungselementes (2),
- Herstellung der Außenform der Beatmungsmaske **durch** Überbetten der Innenform,
- Entfernen des ersten flächigen Materialstücks mit dem Formkörper von der Innenform, wobei das zweite flächige Materialstück als wenigstens ein Versteifungselement (2) weiter an der Außenform anliegt,
- Erneutes Aufsetzen des den Hohlraum abbildenden Formkörpers auf die Innenform und Befüllen des Hohlraums zwischen Innenform und Außenform mit dem Material des Maskenelementes (1) und
- Aushärten des Materials des Maskenelementes (1).

6. Verfahren nach Anspruch 5,
**dadurch gekennzeichnet, dass** vor dem Aufbringen des ersten flächigen Materialstücks die Kontur der Dichtlippe (1b) der herzustellenden Maske in die Innenform eingeformt wird.

7. Verfahren nach Anspruch 5 oder 6,
**dadurch gekennzeichnet, dass** das erste flächige Materialstück aus einem weichen thermoplastischen Material, insbesondere EVA, besteht.

8. Verfahren nach einem der Ansprüche 5 bis 7,
**dadurch gekennzeichnet, dass** das zweite flächige Materialstück als Tiefziehfolie ausgebildet ist.

9. Verfahren nach Anspruch 5 bis 8,
**dadurch gekennzeichnet, dass** das zweite flächige Material aus einem harten thermoplastischen Material, insbesondere PETG, besteht.

10. Verfahren nach einem der Ansprüche 5 bis 9,
**dadurch gekennzeichnet, dass** das erste flächige Materialstück als ein erster und ein zweiter Folienabschnitt ausgebildet ist, wobei zunächst der erste Folienabschnitt als Platzhalter für die innere Dichtlippe (1b) konturgerecht auf die Innenform aufgebracht wird, anschließend der Formkörper auf den ersten Folienabschnitt aufgebracht wird und schließlich der zweite Folienabschnitt als Platzhalter für den Vollkörper (1a) des Maskenelementes (1) konturgerecht auf den ersten Folienabschnitt und den Formkörper aufgebracht wird.

11. Verfahren nach einem der Ansprüche 5 bis 10,
**dadurch gekennzeichnet, dass** der Hohlraum zwischen Innenform und Außenform mit flüssigem Silikon, insbesondere biokompatiblem medical grade 2-Silikon, gefüllt wird.

12. Verfahren nach einem der Ansprüche 5 bis 11,
**dadurch gekennzeichnet, dass** das Aushärten des Materials des Maskenelementes (1) bei Überdruck erfolgt.

13. Verfahren nach Anspruch 12,
**dadurch gekennzeichnet, dass** der Überdruck ca. 2 bar beträgt.

## Claims

1. A respiration mask for oronasal or nasal respiration comprising a flexible mask element (1), which is adapted individually to each patient's facial anatomy, and at least one reinforcing element (2), which is in surface contact with the mask element (1) on the outside, wherein the mask element (1) is designed as a solid body (1a) made of an elastic material,
wherein the mask element (1) comprises an inner peripheral sealing lip (1b), surrounding the nasal or oronasal region, and
wherein the mask element (1) comprises a connecting piece (3) provided for the connection of a respiration tube on its exterior side, wherein
- the elastic material of which the mask element (1) is fabricated is a silicone elastomer,
- the material of the peripheral inner sealing lip (1b) has a lower hardness than the material of the remaining mask element (1), wherein the hardness of the material of the peripheral inner sealing lip (1b) amounts to 10 to 30 Shore, and
- the material of the connecting piece (3) has a greater hardness than the material of the remaining mask element (1), wherein the hardness of the material of the connecting piece (3) amounts to 40 to 70 Shore.

2. The respiration mask according to Claim 1,
**characterized in that** the flexible mask element (1), which is adapted individually to each patient's facial anatomy, is shaped so that it encloses the patient's chin area.

3. The respiration mask according to Claim 1 or 2,
**characterized in that** the mask element (1) has a recess (8) for form-fitting accommodation of the at least one reinforcing element (2) on the outside.

4. The respiration mask according to any one of Claims 1 to 3,
**characterized in that** the at least one reinforcing element (2) has a recess (5) in the region of the bridge of the nose.

5. A method for manufacturing a respiratory mask for nasal or oronasal respiration according to any one of Claims 1 to 4,
**characterized by** the following method steps:
- manufacturing the interior mold of the respiration mask by making an impression of the nasal or oronasal region of the patient,
- applying a first flat piece of material having the correct contour to the interior mold as placeholder for the mask element (1), wherein the piece of material contains a molded body for imaging the cavity (7) surrounded by the sealing lip (1b) of the mask element (1),
- applying a second flat piece of material to the first piece of material to create the at least one reinforcing element (2),
- manufacturing the exterior mold of the respiration mask by bedding over the interior mold,
- removing the first flat piece of material with the molded body from the interior mold, wherein the second flat piece of material is also in contact with the exterior mold as at least one reinforcing element (2),
- renewed placement of the molded body imaging the cavity on the interior mold and filling the cavity between the interior mold and the exterior mold with the material of the mask element (1), and
- curing the material of the mask element (1).

6. The method according to Claim 5,
**characterized in that** the contour of the sealing lip (1b) of the mask to be manufactured is shaped into the interior mold before applying the first flat piece of material.

7. The method according to Claim 5 or 6,
**characterized in that** the first flat piece of material consists of a soft thermoplastic material, in particular EVA.

8. The method according to any one of Claims 5 to 7,
**characterized in that** the second flat piece of material is embodied as a deep-drawn film.

9. The method according to Claims 5 to 8,
**characterized in that** the second flat piece of material consists of a hard thermoplastic material, in particular PETG.

10. The method according to any one of Claims 5 to 9,
**characterized in that** the first flat piece of material is embodied as a first film section and a second film section, wherein the first film section is applied first as a placeholder for the interior sealing lip (1b) to the interior mold with the proper contour, then the molded body is applied to the first film section and finally the second film section is applied to the molded body and to the first film section having the proper contour as a placeholder for the solid body (1a) of the mask element (1).

11. The method according to any one of Claims 5 to 10,
**characterized in that** the cavity between the interior mold and the exterior mold is filled with liquid silicone, in particular biocompatible medical grade-2 silicone.

12. The method according to any one of Claims 5 to 11,
**characterized in that** the material of the mask element (1) is cured under an excess pressure.

13. The method according to Claim 12,
**characterized in that** the excess pressure amounts to approximately 2 bar.

## Revendications

1. Masque respiratoire pour respiration bucco-nasale ou nasale, comprenant un élément masque souple (1) adapté individuellement à l'anatomie du visage du patient et au moins un élément de renfort (2) reposant sur l'extérieur de l'élément masque (1), l'élément masque (1) étant un corps plein (1a) en matériau élastique,
l'élément masque (1) comprenant un rebord d'étanchéité périphérique intérieur (1b) entourant la partie bucco-nasale ou nasale,
et l'élément masque (1) comprenant sur sa face extérieure une pièce de raccordement (3) prévue pour le raccordement d'un tuyau respiratoire,
- le matériau élastique à partir duquel l'élément masque (1) est fabriqué étant un élastomère de silicone,
- le matériau du rebord d'étanchéité périphérique intérieur (1b) ayant une dureté plus faible que le matériau du reste de l'élément masque (1), la dureté du matériau du rebord d'étanchéité périphérique intérieur (1b) étant de 10 à 30 Shore et
- le matériau de la pièce de raccordement (3) ayant une dureté plus élevée que le matériau du reste de l'élément masque (1), la dureté du matériau de la pièce de raccordement (3) étant de 40 à 70 Shore.

2. Masque respiratoire selon la revendication 1,
**caractérisé en ce que** l'élément masque souple (1) adapté individuellement à l'anatomie du visage du patient est formé de manière à entourer la partie du menton du patient.

3. Masque respiratoire selon la revendication 1 ou 2,
**caractérisé en ce que** l'élément masque (1) présente sur sa face extérieure un creux (8) destiné à recevoir par correspondance géométrique l'au moins un élément de renfort (2).

4. Masque respiratoire selon une des revendications 1 à 3,
**caractérisé en ce que** l'au moins un élément de renfort (2) présente un évidement (5) au niveau de l'arête du nez.

5. Procédé de fabrication d'un masque respiratoire pour respiration bucco-nasale ou nasale selon une des revendications 1 à 4,
**caractérisé par** les étapes opératoires suivantes :
- fabrication de la forme intérieure du masque respiratoire par moulage de la partie bucco-nasale ou nasale du patient,
- pose en suivant les contours d'un premier morceau de matériau plaqué sur la forme intérieure et servant d'espace réservé pour l'élément masque (1), le morceau de matériau contenant un corps moulé pour reproduire la cavité (7) circonscrite par le rebord d'étanchéité (1b) de l'élément masque (1),
- pose d'un second morceau de matériau plaqué sur le premier morceau de matériau pour créer l'au moins un élément de renfort (2),
- fabrication de la forme extérieure du masque respiratoire par recouvrement de la forme intérieure,
- retrait du premier morceau de matériau plaqué avec le corps moulé de la forme intérieure, le second morceau de matériau plaqué reposant toujours sous forme d'au moins un élément de renfort (2) sur la forme extérieure,
- repositionnement du corps moulé reproduisant la cavité sur la forme intérieure et remplissage de la cavité entre la forme intérieure et la forme extérieure avec le matériau de l'élément masque (1) et
- durcissement du matériau de l'élément masque (1).

6. Procédé selon la revendication 5,
**caractérisé en ce que**, avant l'application du premier morceau de matériau plaqué, le contour du rebord d'étanchéité (1b) du masque à fabriquer est formé dans la forme intérieure.

7. Procédé selon la revendication 5 ou 6,
**caractérisé en ce que** le premier morceau de matériau plaqué est composé d'une matière thermoplastique molle, en particulier de l'EVA.

8. Procédé selon une des revendications 5 à 7,
**caractérisé en ce que** le second morceau de matériau plaqué se présente sous forme d'une feuille à emboutir.

9. Procédé selon les revendications 5 à 8,
**caractérisé en ce que** le second morceau de matériau plaqué est composé d'une matière thermoplastique rigide, en particulier du PETG.

10. Procédé selon une des revendications 5 à 9,
**caractérisé en ce que** le premier morceau de matériau plaqué se présente sous forme d'une première et d'une seconde section de feuille, la première section de feuille étant d'abord appliquée sur la forme intérieure en suivant les contours pour servir d'espace réservé pour le bord d'étanchéité intérieur (1b), le corps moulé étant ensuite appliqué sur la première section de feuille et la seconde section de feuille étant enfin appliquée sur la première section de feuille et le corps moulé en suivant les contours pour servir d'espace réservé pour le corps plein (1a) de l'élément masque (1).

11. Procédé selon une des revendications 5 à 10,
**caractérisé en ce que** la cavité entre la forme intérieure et la forme extérieure est remplie de silicone liquide, en particulier de silicone biocompatible de qualité médicale 2.

12. Procédé selon une des revendications 5 à 11,
**caractérisé en ce que** le durcissement du matériau de l'élément masque (1) a lieu sous surpression.

13. Procédé selon la revendication 12,
**caractérisé en ce que** la surpression s'élève à environ 2 bars.
